# FASCICULE DE BREVET EUROPEEN

(11) **EP 1 511 448 B1**
(45) Date de publication et mention de la délivrance du brevet: **13.09.2006**
(21) Numéro de dépôt: 03752842.9
(22) Date de dépôt: 20.05.2003
(51) Int. Cl.: A61F 5/44

(54) **POCHE DE RECUEIL DE FLUIDES CORPORELS MUNIE D'UN DISPOSITIF D'OUVERTURE ET DE FERMETURE D'UN CANAL D'EVACUATION**
BEUTEL ZUR AUFNAHME VON KÖRPERFLÜSSIGKEITEN MIT EINER VORRICHTUNG ZUM ÖFFNEN UND SCHLIESSEN EINES ENTLEERUNGSKANALS
BAG FOR COLLECTING BODY FLUIDS PROVIDED WITH A DEVICE FOR OPENING AND CLOSING A DISCHARGE CHANNEL

(30) Priorité: 21.05.2002 FR 0206159
(43) Date de publication de la demande: 09.03.2005
(73) Titulaire: B. Braun Medical SAS, 92100 Boulogne Billancourt (FR)
(72) Inventeur: HOLTERMANN, Henri, F-64500 Saint-Jean-De-Luz (FR)
(74) Mandataire: Arnaud, Jean
(86) Numéro de dépôt international: PCT/FR2003/001526
(87) Numéro de publication internationale: WO 2003/096941

(56) Documents cités:
- WO-A-01/28470
- GB-A- 2 000 683
- US-A- 3 655 118
- US-A- 4 233 977

## Description

L'invention concerne une poche de recueil de fluides corporels munie d'un dispositif d'ouverture et de fermeture d'un canal d'évacuation desdits fluides corporels hors de la poche.

### Domaine technique de l'invention

Après une opération chirurgicale de stomie effectuée suite à une malformation, une tumeur ou une inflammation, et notamment après une iléostomie ou une colostomie, il convient de raccorder l'iléon de l'intestin grêle ou le côlon à la paroi abdominale afin de rétablir les fonctions d'éjection des matières fécales qui ne peuvent plus être réalisées automatiquement et se font donc de manière incontrôlable, au gré de la digestion. Un dispositif hygiénique, confortable et ne perturbant pas les occupations du patient, telles que travail, sport ou loisirs, est donc prévu à cet effet.

Pour cela, le patient stomisé est en général équipé d'une poche de recueil de fluides corporels mise en place immédiatement après l'opération, en général autour d'un orifice abdominal d'évacuation des matières fécales. Cette poche, réalisée classiquement sous la forme d'un sac en matériau tissé souple imperméable au fluide qu'il contient, peut être amovible ou bien son contenu peut être évacué. Dans ce mode particulier de réalisation, il est courant d'utiliser un dispositif de vidange, relié en partie basse de la poche à un col à section réduite, de sorte que les fèces s'écoulent principalement sous l'effet de la pesanteur lorsque le dispositif de vidange est dans une position ouverte.

### Technique antérieure

Différents types de dispositif de vidange de poche de recueil de fluides corporels ont été décrits dans des publications antérieures, et notamment dans le document de brevet US-3 690 320. Ce brevet décrit un dispositif de vidange dans lequel la partie de section réduite de la poche, ou canal d'évacuation, est divisée en trois secteurs séparés les uns des autres par deux pliures et maintenus en position par des bandes de matériau à boucles et crochets, tel que "Velcro". L'ouverture est réalisée dès que les bandes sont détachées, et elle est donc peu pratique et peu fiable car les bandes peuvent se détacher toutes seules, notamment si elles s'accrochent à des vêtements.

Il est également déjà connu du document de brevet US-3 825 005 de réaliser un dispositif de vidange dans lequel les opérations d'ouverture et de fermeture s'effectuent en deux étapes successives avec une position intermédiaire dans laquelle il est nécessaire de prévoir un premier moyen de fermeture lorsqu'un second moyen est ouvert, afin d'éviter une ouverture intempestive et des fuites.

Or, dans ce dispositif, le maintien à l'état fermé des premiers moyens de fermeture dans la position intermédiaire du dispositif de vidange nécessite un renforcement mécanique des films du canal d'évacuation, afin de résister à la pression s'exerçant sur un pli formant une zone de fermeture où s'accumulent les selles fluides à évacuer, des plaques de renfort sont utilisées sur le canal d'évacuation. Ces plaques sont gênantes pour le patient qui porte la poche, rendent le dispositif de vidange plus complexe à réaliser, et peuvent même nuire à la fiabilité de l'étanchéité du dispositif de vidange dans certaines positions du patient.

Le document WO 01 28 470 décrit une poche de stomie qui comporte une languette fixée à une paroi de la poche et qui vient se rabattre sur un canal plié d'évacuation.

Le document US-4 233 977 décrit une poche de stomie ayant une bande relativement rigide qui délimite avec un côté de la poche un logement dans lequel est introduit un canal d'évacuation plié.

### Exposé de l'invention

Le but de l'invention est donc de résoudre les inconvénients évoqués ci-dessus.

Pour ce faire, l'invention propose de réaliser un dispositif de vidange dans lequel les opérations d'ouverture et de fermeture s'effectuent en deux étapes successives, avec une position intermédiaire dans laquelle une des portions du canal d'évacuation est maintenue dans un état plié, et le canal d'évacuation est dépourvu de renfort.

Plus précisément, l'invention se rapporte à une poche de recueil de fluides corporels, tels que des matières fécales, destinée à être disposée sur l'abdomen d'un patient ayant subi une stomie, selon la revendication 1

Selon différents modes de réalisation préférés de l'invention :
- l'organe de rigidification est sensiblement perpendiculaire au canal d'évacuation et il se présente sous la forme d'un passant en matière plastique plus rigide que la matière constituant les films du sac et des parois du canal d'évacuation,
- le second moyen d'ouverture/fermeture comprend au moins un pli transversal du canal d'évacuation obturant celui-ci dans la position fermée du dispositif d'ouverture/fermeture et en outre un second pli disposé au ras du col, en bordure de l'organe de rigidification, quelle que soit la position ouverte ou fermée du dispositif d'ouverture/fermeture,
- elle comprend une languette de manipulation fixée au second pli du second moyen d'ouverture/fermeture, qui passe par la fente située entre l'organe de rigidification et le film de la poche, et qui dépasse de cet organe pour pouvoir être saisie, de sorte que le second moyen d'ouverture/fermeture peut être déplacé de sa position d'ouverture à sa position de fermeture par tirage de la languette,
- le premier moyen d'ouverture/fermeture est disposé, en position fermée, par dessus l'organe de rigidification et il comprend une bande auto-agrippante, présentant au moins une première partie, par exemple une bande de type "Velcro" mâle ou femelle ou tout type d'adhésif repositionnable, disposée sur la languette de manipulation située sensiblement à l'opposé de l'orifice de vidange du canal d'évacuation,
- la languette de manipulation est reliée à un pli du second moyen d'ouverture/fermeture, et
- l'organe de rigidification a un prolongement du côté opposé de l'ouverture de la fente située entre l'organe de rigidification et le film de la poche du côté du col de la poche, à pratiquement à l'emplacement de coopération des partie de bande auto-aggripante, et/ou
- le canal d'évacuation est muni, à l'écart des moyens d'ouverture/fermeture, de plis transversaux facilitant son pliage et son dépliage.

### Description sommaire des dessins

D'autres caractéristiques, détails et avantages de l'invention ressortiront à la lecture de la description faite en référence aux dessins annexés donnés à titre d'exemple et dans lesquels :
- la figure 1, une vue de face d'une poche de recueil de matières fécales conforme à l'invention et munie d'un canal d'évacuation dans un état totalement fermé,
- la figure 2, une vue de côté de la figure 1 ;
- la figure 3, une vue de détail de la figure 1 montrant des premier et second moyens de fermeture du canal d'évacuation dans une position totalement fermée ;
- la figure 4, une vue de face de la poche avec le premier moyen de fermeture en position dépliée ouverte,
- la figure 5, une vue de côté de la figure 4,
- la figure 6, une vue de face de la poche avec le canal d'évacuation en position intermédiaire d'ouverture,
- la figure 7, une vue de côté de la figure 6,
- la figure 8, une vue de face de la poche avec le canal d'évacuation en position totalement dépliée, c'est-à-dire avec le second moyen de fermeture ouvert,
- la figure 9, une vue de côté de la figure 8,
- la figure 10, une vue analogue à la figure 8, mais représentant une variante, et
- les figures 11 et 12 des schémas indiquant la position des différents éléments de la variante de la figure 10.

### Description détaillée de modes de réalisation

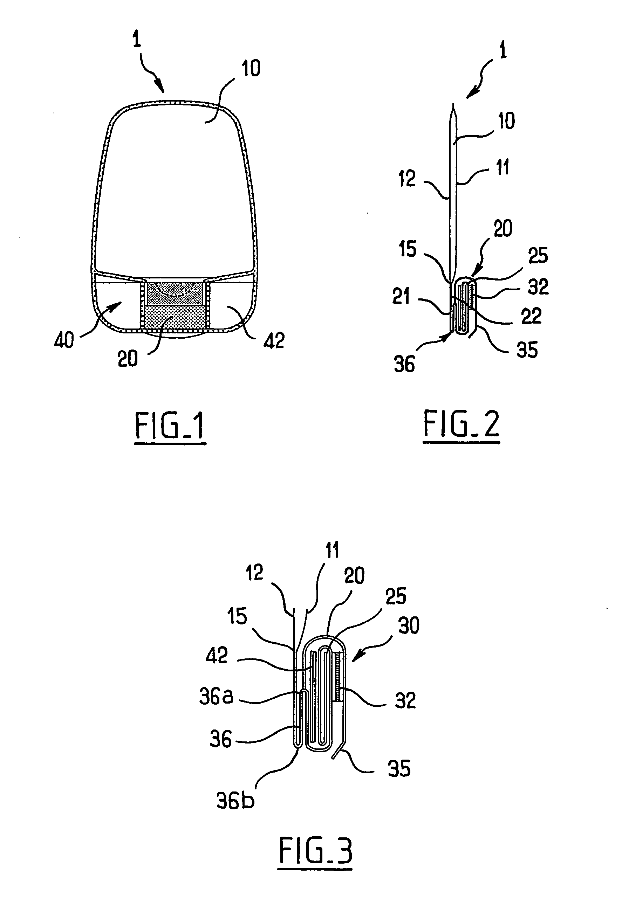

Les figures 1 à 3 représentent, dans un état fermé, une poche 1 de recueil de matières fécales fluides s'écoulant à partir d'un orifice (non représenté) réalisé sur l'abdomen d'un patient stomisé suite par exemple à une iléostomie.

Cette poche 1 comprend un sac 10 constitué par deux films fins 11 et 12 espacés et soudés l'un à l'autre à leur périphérie de manière étanche aux matières fécales. Le sac 10 comprend également un col ouvert 15 se prolongeant par un canal allongé 20 d'évacuation des matières fécales hors du sac 10 lorsqu'il est nécessaire de vidanger la poche de recueil 1.

Le canal d'évacuation 20 est constitué par deux films réalisés dans le même matériau que ceux du sac, typiquement des films à barrière d'odeurs, comme par exemple des films composites vendus sous la marque "Saranex". Tout autre film, réalisé à partir de polyéthylène, de PVC (chlorure de polyvinyle), de PVDC (chlorure de polyvinylidène), d'EVA (résine d'acétate de vinyl-éthylène), ou tout polymère extrudable sous forme de film, peut également convenir. Ces films forment deux parois 21 et 22 espacées et soudées longitudinalement l'une à l'autre par leurs bords respectifs de manière étanche aux matières fécales. Le canal d'évacuation 20 se termine, à une extrémité opposée au col 15, par un orifice de vidange 25 permettant le passage des matières fécales lors de la vidange de la poche 1.

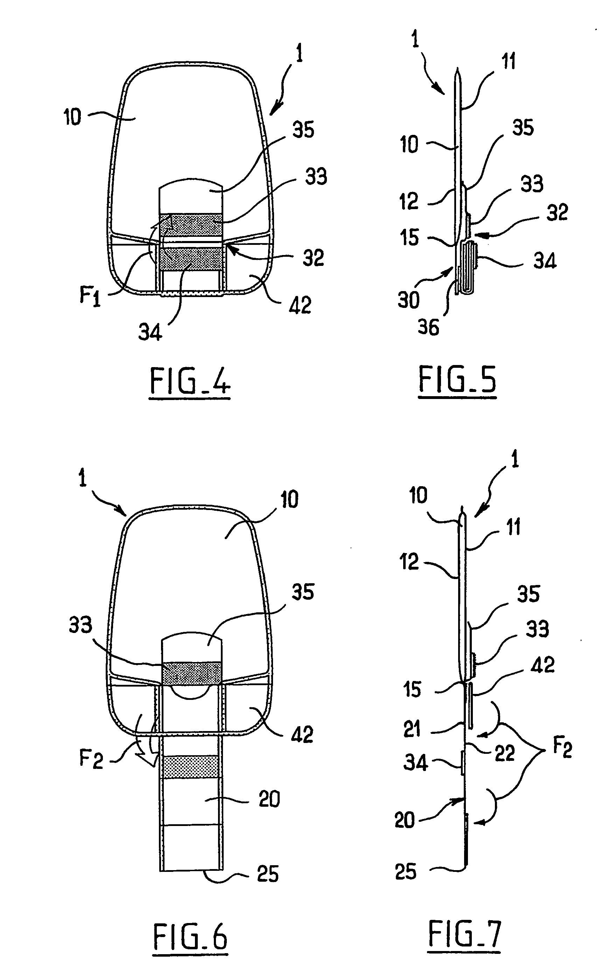

La poche 1 est munie d'un dispositif 30 d'ouverture/fermeture du canal d'évacuation 20 actionnable par l'utilisateur qui est équipé de ladite poche 1 afin qu'il puisse la vidanger tout seul très facilement. A cet effet, le dispositif 30 d'ouverture/fermeture comprend un premier moyen 32 d'ouverture/fermeture du canal d'évacuation 20. Ce premier moyen d'ouverture/fermeture 32 prend dans cet exemple la forme d'une bande auto-agrippante vendue sous la marque déposée "Velcro" dont une partie mâle 33 est disposée sur la face interne d'une languette de manipulation 35 située, comme cela est représenté sur les figures 6 à 9, sensiblement à l'opposé de l'orifice de vidange 25 du canal d'évacuation 20, dans l'état déplié ouvert dudit canal 20. L'autre partie 34 femelle du premier moyen 32 d'ouverture/fermeture est disposée sur une surface externe d'une paroi 22 du canal d'évacuation, transversalement à celui-ci.

Le dispositif d'ouverture/fermeture 30 comprend également un second moyen 36 d'ouverture/fermeture matérialisé par un premier et un second plis 36a et 36b. disposés transversalement par rapport au canal d'évacuation 20. La languette 35 est reliée au premier pli 36a qui est le plus éloigné du col 15 du sac 10 tandis que le second pli 36b est disposé plus près du col 15.

Le sac 10 de la poche 1 est par ailleurs munie d'un organe de rigidification 40 à proximité du col 15. Cet organe se présente sous la forme d'un passant 42, semblable à un passant de ceinture, soudé et/ou collé extérieurement au film 11 du sac 10 sur sensiblement toute la largeur dudit sac 10. Ce passant 42 est réalisé dans un matériau plastique plus solide que la matière constituant le sac 10 et le canal d'évacuation 20, par exemple sous forme d'une feuille de polyéthylène, d'un film composite de PE/PET (polyéthylène-téréphtalate de polyéthylène), d'un film de PVC rigide ou d'un film PET. Ce passant 42 délimite, avec le film 11, une fente 45 perpendiculaire au canal d'évacuation 20 et dans laquelle est disposé le second moyen 36 d'ouverture/fermeture dans l'état fermé replié du dispositif d'ouverture/fermeture 30, comme cela est visible sur la vue de détail de la figure 3.

Les figures 4 à 7 illustrent une première étape de l'ouverture du canal d'évacuation 20. Cette première étape consiste à détacher la bande du premier moyen d'ouverture/fermeture 32 qui est placé, dans son état fermé, par dessus le passant 42. Pour cela, l'utilisateur lève la languette de manipulation 35, comme illustré par la flèche F1 de la figure 4. Le résultat de cette manoeuvre est illustré par les figures 4 et 5 sur lesquelles la bande 32 est représentée dans son état ouvert.

Suite à cette opération, le canal d'évacuation 20 peut être déplié par l'utilisateur pour atteindre une position intermédiaire représentée par les figures 6 et 7 et par la flèche F2. Dans cette position intermédiaire, le canal d'évacuation 20 est encore maintenu fermé par les seconds moyens d'ouverture/fermeture 36 dont les plis 36a et 36b sont maintenus en position par le passant 42.

Il suffit alors de tirer légèrement sur l'extrémité du canal d'évacuation 20, de préférence près de l'orifice de vidange 25, pour déplier les plis 36a et 36b disposés en accordéon sous le passant 42 afin de permettre le passage des matières fécales bloquées par ces plis pour qu'elles s'écoulent dans le canal d'évacuation 20 et sortent par l'orifice de vidange 25. Dans cette position dépliée ouverte, la languette de manipulation 35 est également sous le passant mais reste-accessible.

Pour refermer le canal d'évacuation 20, il suffit de tirer sur la languette pour replier le second moyen d'ouverture/fermeture 36 afin que les plis 36a et 36b se remettent en place en accordéon sous le passant 42.

Ensuite, le canal est replié de manière à positionner les parties mâle et femelle 33 et 34 du premier moyen d'ouverture/fermeture 32 l'une en face de l'autre pour fermer complètement le canal d'évacuation 20. A cet effet, le canal d'évacuation 20 est muni, à l'écart des moyens d'ouverture/fermeture 32 et 36, de plis transversaux 26, 27 et 28 facilitant son pliage et son dépliage.

La poche de recueil de fluide corporel 1 selon la présente invention procure ainsi un haut niveau de sécurité au niveau de l'étanchéité du canal d'évacuation 20, même lorsque celui-ci est partiellement ouvert, et, une grande facilité d'utilisation, grâce à l'utilisation du passant de rigidification 42 qui maintient en place le second moyen 36 d'ouverture/fermeture.

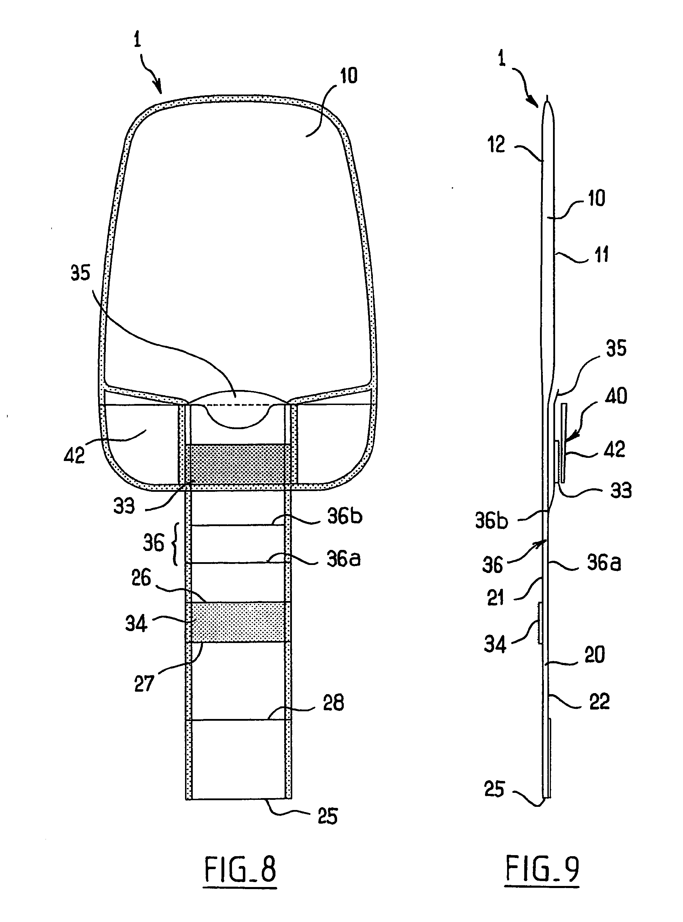

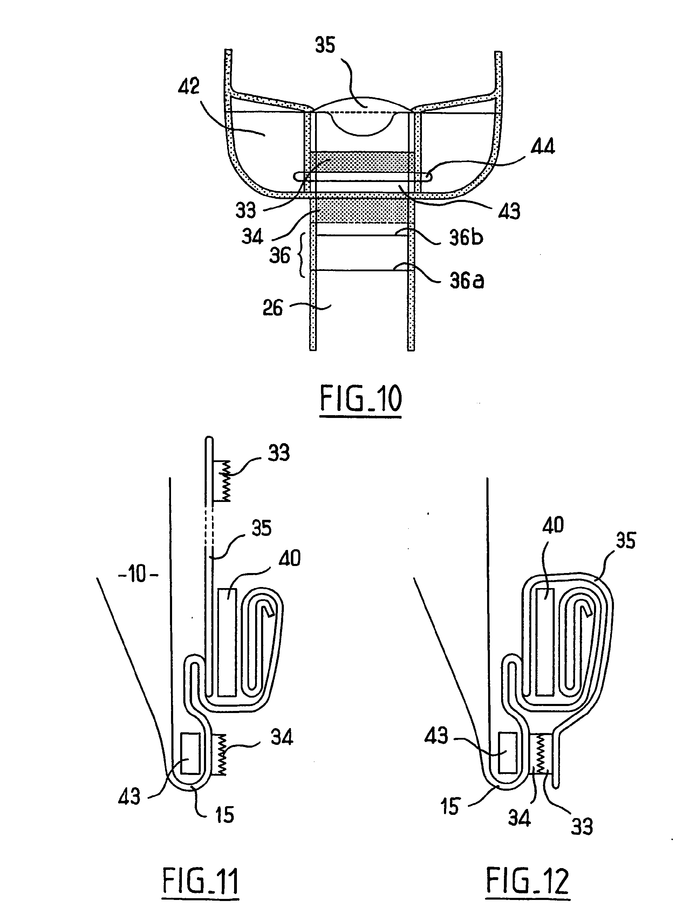

Les figures 10 à 12 représentent une variante comportant des caractéristiques différentes de celles du mode de réalisation des figures 8 et 9.

Dans ce mode de réalisation, la partie 34 de bande auto-agrippante est au-dessus du pli 36b auquel est soudé la languette 35, c'est-à-dire plus prèss du col 15. La partie 33 de bande auto-agrippante fixée à la languette 35 entoure plus complètement le canal d'évacuation et le maintient plus fermement que dans le mode de réalisation des figures 1 à 3, comme l'indique notamment la comparaison des figures 3 et 12.

On a aussi représenté un prolongement 43 de l'organe 40 de rigidification, séparé de la partie principale de cet organe par une fente 44 dans laquelle passent le canal d'évacuation plié et la languette. Ce prolongement constitue un support rigide qui soutient les parties de la bande auto-agrippante lorsqu'elle sont appliquées l'une sur l'autre à la fermeture du premier moyen d'ouverture/fermeture.

Il doit être bien entendu toutefois que ces exemples sont donnés uniquement à titre d'illustration de l'objet de l'invention dont ils ne constituent en aucune manière une limitation. En particulier, cette poche peut servir dans des cas de colostomie. La bande de type "Velcro" peut être remplacée par une bande munie d'un adhésif repositionnable. Le moyen de rigidification 42 peut être réalisé sous forme de plusieurs petites bandes rigides légèrement espacées afin d'augmenter le confort d'utilisation de la poche.

## Revendications

1. Poche (1) de recueil de fluides corporels, tels que des matières fécales, destinée à être disposée sur l'abdomen d'un patient ayant subi une stomie et comprenant :
- un sac (10) étanche au fluide formé par deux films (11, 12) liés l'un à l'autre à leur périphérie et se terminant par un col (15),
- un canal (20) allongé d'évacuation relié audit col (15) et formé par deux parois (21, 22) liées l'une à l'autre longitudinalement de manière étanche au fluide, le canal d'évacuation (20) présentant un orifice de vidange (25) situé à une extrémité opposée au col (15) pour l'écoulement des fluides corporels en dehors de la poche (1) et,
- un dispositif d'ouverture/fermeture (30) pour faire passer le canal d'évacuation (20) d'un état fermé replié à un état ouvert déplié, le dispositif d'ouverture/fermeture (30) comprenant un premier (32) et un second (36) moyens d'ouverture/fermeture aménagés transversalement par rapport au canal d'évacuation (20), lesdits moyens d'ouverture/fermeture (32, 36) étant tous les deux actifs dans l'état fermé du canal d'évacuation (20), **caractérisée en ce que** ledit dispositif d'ouverture/fermeture (30) est adapté pour ouvrir le canal d'évacuation (20) par mise en oeuvre, selon deux étapes successives, du premier (32) puis du second (36) moyen d'ouverture/fermeture, un des films (11, 12) du sac (10) étant muni extérieurement d'un organe de rigidification (40) disposé à proximité du col (15), le second moyen d'ouverture/fermeture (36) du canal d'évacuation (20) comprenant, dans son état fermé replié, au moins deux plis (36a) et (36b) obturant le canal (20), disposés en accordéon et obtenus par insertion d'au moins une partie du second moyen d'ouverture/fermeture dans une fente (45) située entre l'organe de rigidification (40) et le film (11, 12) du sac (10), et un pli (36a) des seconds moyens d'ouverture/fermeture (36) est disposé transversalement par rapport au canal d'évacuation (20)

2. Poche selon la revendication 1, **caractérisée en ce que** l'organe de rigidification (40) se présente sous la forme d'un passant (42) de matière plastique plus rigide que la matière constituant les films (11, 12) du sac et des parois (21, 22) du canal d'évacuation (20).

3. Poche selon l'une des revendications 1 et 2 **caractérisée en ce que** le second pli (36b) des moyens d'ouverture/fermeture (36) est disposé plus près du col (15) et de l'organe de rigidification (40) que le premier pli (36a), quelle que soit la position ouverte ou fermée du dispositif d'ouverture/fermeture (30).

4. Poche selon l'une quelconque des revendications précédentes, **caractérisée en ce que** le premier moyen d'ouverture/fermeture (32) est disposé, en position fermée, par dessus l'organe de rigidification (40).

5. Poche selon l'une quelconque des revendications précédentes, **caractérisée en ce qu'**elle comprend une languette de manipulation (35) fixée sur le canal d'évacuation au niveau du second pli (36a) du second moyen d'ouverture/fermeture (36), au moins partiellement insérée entre l'organe de rigidification et le film (11, 12) de la poche (15) en position dépliée du dispositif d'ouverture/fermeture, et qui dépasse de cet organe, de sorte que le second moyen d'ouverture/fermeture (36) peut être déplacé de sa position d'ouverture à sa position de fermeture par tirage de la languette (35).

6. Poche selon la revendication 5, **caractérisée en ce que** l'organe de rigidification (40) est muni d'une fente (44) sensiblement perpendiculaire au canal d'évacuation (20) par laquelle passe la languette (35) et dans laquelle est introduit le second moyen de fermeture/ouverture (36), préalablement à l'insertion partielle de celui-ci entre l'organe de rigidification (40) et le film (11 ; 12) du sac (10) lors de la fermeture de la poche.

7. Poche selon l'une des revendications 5 et 6, **caractérisée en ce que** le premier moyen d'ouverture/fermeture (32) comprend une bande auto-agrippante présentant au moins une première partie coopérante (33) disposée sur la languette (35) de manipulation et une seconde partie coopérante (34) située sur le canal d'évacuation (20).

8. Poche selon la revendication 7, **caractérisée en ce que** l'organe de rigidification (40) est muni d'un prolongement (43) au delà de la fente (44) dans la direction du col (15) de la poche, situé au moins partiellement entre le film (11 ; 12) du sac (10) et les parties (33,34) coopérantes de bande auto-agrippantes dans la position fermée de la poche.

9. Poche selon l'une quelconque des revendications précédentes, **caractérisée en ce que** le canal d'évacuation (20) est muni, à l'écart des moyens d'ouverture/fermeture (32, 36), de plis transversaux (26, 27, 28), facilitant son pliage et son dépliage.

## Claims

1. A bag (1) for collecting body fluids, such as faecal material, designed to be disposed on the abdomen of a patient having undergone an ostomy and comprising:
- a fluid-tight pouch (10) formed of two films (11, 12) joined together at their periphery and ending in a neck (15),
- an elongate discharge channel (20) connected to said neck (15) and formed of two walls (21, 22) joined together longitudinally in fluid-tight manner, the discharge channel (20) having a drainage orifice (25) situated at the opposite end from the neck (15) for body fluids to flow out of the bag (1) , and
- an opening/closing device (30) for transferring the discharge channel (20) from a folded closed state to an unfolded open state, the opening/closing device (30) comprising a first (32) and a second (36) opening/closing means provided transversely relative to the discharge channel (20), said opening/closing means (32, 36) both being active when the discharge channel (20) is in the closed state, **characterised in that** said opening/closing device (30) is adapted to open the discharge channel (20) by operation, in two successive stages, of the first (32) and then the second (36) opening/closing means, one of the films (11; 12) of the pouch (10) being provided on the outside with a stiffening member (40) disposed in the vicinity of the neck (15), the second opening/closing means (36) of the discharge channel (20) comprising, in its folded, closed state, at least two folds (36a) and (36b) closing off the channel (20), disposed in the manner of a concertina and obtained by insertion of at least one part of the second opening/closing means into a slot (45) situated between the stiffening member (40) and the film (11; 12) of the pouch (10), and one fold (36a) of the second opening/closing means (36) being disposed transversely relative to the discharge channel (20).

2. A bag according to claim 1, **characterised in that** the stiffening member (40) takes the form of a loop (42) of stiffer plastics material than the material constituting the films (11; 12) of the pouch and of the walls (21, 22) of the discharge channel (20).

3. A bag according to either one of claims 1 and 2, **characterised in that** the second fold (36b) of the opening/closing means (36) is disposed nearer to the neck (15) and the stiffening member (40) than the first fold (36a), whatever the open or closed position of the opening/closing device (30).

4. A bag according to any one of the preceding claims, **characterised in that** the first opening/closing means (32) is disposed, in the closed position, over the stiffening member (40).

5. A bag according to any one of the preceding claims, **characterised in that** it comprises a handling tongue (35) fixed to the discharge channel at the level of the second fold (36a) of the second opening/closing means (36), said tongue being at least partially inserted between the stiffening member and the film (11; 12) of the bag (15) in the unfolded position of the opening/closing device, and extending beyond this member, such that the second opening/closing means (36) may be displaced from its open position to its closed position by pulling on the tongue (35) .

6. A bag according to claim 5, **characterised in that** the stiffening member (40) is provided with a slot (44) substantially perpendicular to the discharge channel (20), through which slot the tongue (35) passes and into which there is introduced the second opening/closing means (36), prior to partial insertion thereof between the stiffening member (40) and the film (11; 12) of the pouch (10) at the time of closure of the bag.

7. A bag according to either one of claims 5 and 6, **characterised in that** the first opening/closing means (32) comprises a self-gripping strip having at least a first, cooperating part (33) disposed on the handling tongue (35) and a second, cooperating part (34) situated on the discharge channel (20).

8. A bag according to claim 7, **characterised in that** the stiffening member (40) has an extension (43) beyond the slot (44) in the direction of the neck (15) of the bag, situated at least partially between the film (11; 12) of the pouch (10) and the cooperating parts (33, 34) of the self-gripping strip when the bag is in the closed position.

9. A bag according to any one of the preceding claims, **characterised in that** the discharge channel (20) is provided, away from the opening/closing means (32, 36), with transverse folds (26, 27, 28) facilitating folding and unfolding thereof.

## Patentansprüche

1. Beutel (1) zur Aufnahme von Körperflüssigkeiten, wie z. B. Fäkalien, der dazu bestimmt ist, auf dem Abdomen eines Patienten, der sich einer Stomaoperation unterzogen hat, angeordnet zu werden, und der aufweist:
- einen gegen Fluid dichten Sack (10), der aus zwei Folien (11, 12) gebildet ist, die an ihrem Umfang miteinander verbunden sind und in einem Kragen (15) enden,
- einen länglichen Entleerungskanal (20), der mit dem Kragen (15) verbunden und durch zwei Wände (21, 22) gebildet ist, die längs miteinander in dichter Weise gegen Flüssigkeit verbunden sind, wobei der Entleerungskanal (20) eine Entleerungsöffnung (25) aufweist, die an einem dem Kragen (15) gegenüberliegenden Ende zum Fließen der Körperflüssigkeiten aus dem Beutel (1) nach außen angeordnet ist, und
- eine Öffnungs-/Schließvorrichtung (30), um den Entleerungskanal (20) aus einem gefalteten, geschlossenen Zustand in einen entfalteten, offen Zustand übergehen zu lassen, wobei die Öffnungs-/Schließvorrichtung (30) ein erstes (32) und ein zweites (36) Öffnungs-/Schließmittel aufweist, die quer bezüglich des Entleerungskanals (20) eingerichtet sind, wobei die Öffnungs-/Schließmittel (32, 36) beide in dem geschlossenen Zustand des Entleerungskanals (20) aktiv sind, **dadurch gekennzeichnet, daß** die Öffnungs-/Schließvorrichtung (30) geeignet ist, den Entleerungskanal (20) durch die Anwendung gemäß zweier aufeinanderfolgender Schritte des ersten (32) dann des zweiten (36) Öffnungs-/Schließmittels zu öffnen, wobei eine der Folien (11, 12) des Sackes (10) außen mit einem Versteifungsorgan (40) ausgestattet ist, das in der Nähe des Kragens (15) angeordnet ist, und das zweite Öffnungs-/Schließmittel (36) des Entleerungskanals (20) in seinem eingefalteten, geschlossenen Zustand mindestens zwei Falten (36a und 36b) aufweist, welche den Kanal (20) verschließen, die ziehharmonikaartig angeordnet sind und durch Einführen mindestens eines Teils des zweiten Öffnungs-/Schließmittels in einen Schlitz (45) erhalten werden, der zwischen dem Versteifungsorgan (40) und der Folie (11, 12) des Sackes (10) liegt, und eine Falte (36a) des zweiten Öffnungs-/Schließmittels (36) quer bezüglich des Entleerungskanals (20) angeordnet ist.

2. Beutel nach Anspruch 1, **dadurch gekennzeichnet, daß** sich das Versteifungsorgan (40) in der Form einer Schlaufe (42) aus Kunststoffmaterial darstellt, das steifer ist als das Material, welches die Folien (11, 12) des Beutels und der Wände (21, 22) des Entleerungskanals (20) bildet.

3. Beutel nach einem der Ansprüche 1 und 2, **dadurch gekennzeichnet, daß** die zweite Falte (36b) der Öffnungs-/Schließmittel (36) näher an dem Kragen (15) und dem Verstärkungsorgan (40) angeordnet ist als die erste Falte (36a), wie auch immer die geöffnete oder geschlossene Position der Öffnungs-/Schließvorrichtung (30) ist.

4. Beutel nach einem der vorhergehenden Ansprüche, **dadurch gekennzeichnet, daß** das erste Öffnungs-/Schließmittel (32) in geschlossener Position über dem Verstärkungsorgan (40) angeordnet ist.

5. Beutel nach einem der vorhergehenden Ansprüche, **dadurch gekennzeichnet, daß** er eine Betätigungszunge (35) aufweist, die auf dem Entleerungskanal auf der Höhe der zweiten Falte (36a) des zweiten Öffnungs-/Schließmittels (36) befestigt, mindestens teilweise zwischen das Verstärkungsorgan und die Folie (11, 12) des Sackes (15) in entfalteter Position der Öffnungs-/Schließvorrichtung eingeführt ist und welche dieses Organ derart überragt, daß das zweite Öffnungs-/Schließmittel (36) aus seiner Öffnungsposition in seine Verschließposition durch Ziehen der Zunge (35) verschoben werden kann.

6. Beutel nach Anspruch 5, **dadurch gekennzeichnet, daß** das Versteifungsorgan (40) mit einem Schlitz (44) versehen ist, der sich im wesentlichen senkrecht zu dem Entleerungskanal (20) befindet, durch welchen die Zunge (35) hindurchgeht und in welchen das zweite Schließ-/Öffnungsmittel (36) vor dem teilweisen Einführen desselben zwischen das Versteifungsorgan (40) und die Folie (11;12) des Sackes (10) während des Schließens des Beutels eingeführt wird.

7. Beutel nach einem der Ansprüche 5 und 6, **dadurch gekennzeichnet, daß** das erste Öffnungs-/Schließmittel (32) ein selbstgreifendes Band aufweist, das mindestens einen ersten zusammenwirkenden Teil (33) hat, der auf der Betätigungszunge (35) angeordnet ist, und einen zweiten zusammenwirkenden Teil (34), der auf dem Entleerungskanal (20) liegt.

8. Sack nach Anspruch 7, **dadurch gekennzeichnet, daß** das Versteifungsorgan (40) mit einer Verlängerung (43) oberhalb des Schlitzes (44) in der Richtung des Kragens (15) des Beutels versehen ist, die mindestens teilweise zwischen der Folie (11; 12) des Sackes (10) und den selbstergreifenden, zusammenwirkenden Teilen (33, 34) des Bandes in der geschlossenen Position des Beutels angeordnet ist.

9. Beutel nach einem der vorhergehenden Ansprüche, **dadurch gekennzeichnet, daß** der Entleerungskanal (20) im Abstand von den Öffnungs-/Schließmitteln (32, 36) mit Querfalten (26, 27, 28) versehen ist, die sein Falten und sein Entfalten erleichtern.
